# EUROPÄISCHE PATENTSCHRIFT

(11) **EP 0 634 432 B1**
(45) Veröffentlichungstag und Bekanntmachung des Hinweises auf die Patenterteilung: **28.01.1998**
(21) Anmeldenummer: 94107293.6
(22) Anmeldetag: 10.05.1994
(51) Int. Cl.: C08G 18/40, C08G 18/66

(54) **Polyurethan-Formmasse und daraus hergestellte, biologisch abbaubare Filamente**
Polyurethane moulding material and the biodegradable filaments prepared therefrom
Matériau de moulage de polyuréthane et les filaments biodégradable à partir de celui-ci

(30) Priorität: 11.06.1993 DE 4319439
(43) Veröffentlichungstag der Anmeldung: 18.01.1995
(73) Patentinhaber: Firma Carl Freudenberg, 69469 Weinheim (DE)
(72) Erfinder: Mühlfeld, Horst, D-64689 Grasellenbach (DE); Klein, Lieselotte, Dr., D-68167 Mannheim (DE); Groten, Robert, Dr., D-69469 Weinheim (DE)

(56) Entgegenhaltungen:
- EP-A- 0 451 786
- WO-A-91/18036
- US-A- 4 379 904

## Beschreibung

Die Erfindung befaßt sich mit biologisch abbaubaren und thermoplastisch verarbeitbaren, elastischen, insbesondere mit der Schmelzspinntechnik herstellbaren Polyurethan-Filamenten. Ferner wird deren Verwendung angegeben.

Vliesstoffe aus elastischen Spinnfilamenten sind wegen ihrer hervorragenden Festigkeitseigenschaften und ihres guten Tragekomforts Hauptbestandteile von Inkontinenzartikeln, z.B. von Windeln und Damenbinden.

Da es sich um Wegwerfprodukte handelt, muß der biologischen Abbaubarkeit solcher Filamentvliesstoffe hohe Aufmerksamkeit geschenkt werden. Der hervorragenden Verspinnbarkeit linearer, d.h. aus rein aliphatischen Komponenten aufgebauter, Polyurethan-Formmassen zu Filamenten mit gerade für die oben genannten Artikel hervorragenden Elastizitäts- und Festigkeitseigenschaften steht jedoch der Nachteil einer nicht befriedigenden biologischen Abbaubarkeit entgegen. So beschreibt zwar DE 42 03 307 C1 eine thermoplastisch in Form von Sinterpulver bearbeitbare Polyurethan-Formmasse zur Herstellung von genarbten Sinterfolien für Armaturenbretter. Diese Formmasse ist ausschließlich aus linearen Komponenten herstellbar, nämlich aus 100 Gew.-Teilen eines Polyolgemisches aus einem aliphatischen Polycarbonatdiol und einem Polyesterdiol auf Basis Adipinsäure, Hexandiol und Neopentylglykol, und aus Hexamethylendiisocyanat. Als Kettenverlängerer dient 1,4-Butandiol. Die NCO-Kennzahl, gebildet aus dem mit 100 multiplizierten Quotienten der Äquivalenzverhältnisse von Isocyanatgruppen und der Summe aller in der Formmasse enthaltenen Hydroxylgruppen, einschließlich derjenigen der Kettenverlängerer, liegt bei 97 bis 99, was einen Unterschuß an Diisocyanat bedeutet.

Über die Spinnbarkeit dieser Formmasse zu kontinuierlichen Filamenten ist nichts ausgesagt. Versuche ergaben zwar, daß die Masse aufgrund ihrer niedrigviskosen Schmelze auch zu Filamenten verarbeitet werden kann. Solche Polyurethane sind aufgrund ihres hohen Anteils an Polycarbonat im molekularen Gerüst besonders alterungsbeständig.

Reine Polyesterpolyurethane.mit der Polyolkomponente auf Basis Adipinsäure und Glykol werden, wenn keine zusätzlichen Schutzmittel eingesetzt werden, durch einwirkende Feuchtigkeit hydrolytisch abgebaut. Die Polyesterkomponente im Weichsegment wird durch Wasser verseift, und die Polyurethanketten spalten in kürzere Einheiten auf. Dieser Abbau erfolgt bereits unter milden Bedingungen, d.h. bei Temperaturen und einer Luftfeuchtigkeit, welche etwa den klimatischen Verhältnissen unserer Breitengrade entsprechen.

Untersuchungen zeigten eine Verringerung der Festigkeit von Polyesterpolyurethanen um 50% nach einer Lagerzeit von 4 Jahren bei Normklima (23°C, 50% relative Feuchte). Offenbar sind die entstehenden niedermolekulare Spaltprodukte, überwiegend Polyurethan-Hartsegmente, gegen einen weiteren abbauenden Einfluß von Feuchtigkeit und Sauerstoff sehr stabil.

Die Aufgabe der vorliegenden Erfindung besteht darin, den Aufbau einer thermoplastischen, nur aus aliphatischen Komponenten bestehenden Polyurethan-Formmasse anzugeben, welche aufgrund ihrer Linearität zu elastischen Filamenten aus der Schmelze verspinnbar ist und welche, im Gegensatz zu bekannten Formmassen dieser Art, eine solche biologische Abbaubarkeit aufweist, daß die daraus hergestellten Produkte bei Deponielagerung in der ersten Abbaustufe durch Hydrolyse und in einem weiteren Abbauschritt durch Enzyme und Mikroorganismen innerhalb weniger Wochen vollständig zerfallen sind.

Die Lösung dieser Aufgabe besteht in einer Polyurethan-Formmasse, welche die Kennzeichen des ersten Patentanspruchs aufweist, sowie daraus ersponnenen Filamenten.

Die Forderung nach Thermoplastizität und Verspinnbarkeit aus der Schmelze zu gleichförmigen, elastischen, festen Filamenten wird erfüllt durch die an sich bekannte Maßnahme, die Formmasse aus rein linearen, d.h. ausschließlich aliphatischen Komponenten, zu synthetisieren. Es gab jedoch keinen Hinweis aus dem Stand der Technik, wie ein solcher Aufbau gezielt zu erfolgen habe, um die in der Aufgabenstellung geforderte biologische Abbaubarkeit zu erzielen. Es sind bisher auch keine thermoplastischen Polyurethan-Formmassen bekannt geworden, deren Eignung zur Verspinnbarkeit zu elastischen, biologisch abbaubaren Filamenten erkannt oder gefordert worden wäre.

Das Polyolgemisch zur erfindungsgemäßen Darstellung der Filamente besteht zum einen aus 70 bis 90 Gew.-Teilen Polyesterpolyol mit einem Molekulargewicht von 2000, welches auf der Basis von Adipinsäure mit Ethandiol oder mit Butandiol, Hexandiol, Diethylenglykol oder Neopentylglykol beruht. Zum anderen besteht das Polyolgemisch aus 10 bis 30 Gew.-Teilen Polyethylenglykol mit einem Molekulargewicht von 800 bis 4000.

In einem Äquivalenzverhältnis von 2,8 : 1,0 bis 12,0 : 1,0 zum Polyolgemisch werden ferner 1,6-Hexamethylendiisocyanat, Isophorondiisocyanat oder Dicyclohexylmethan-4,4'-Diisocyanat umgesetzt.

Als Kettenverlängerer dienen 1,4-Butandiol oder 1,6-Hexandiol, alternativ oder in Kombination, in einem Äquivalenzverhältnis zum Polyolgemisch von 1,75 : 1,0 bis 11,3 : 1,0.
Die NCO-Kennzahl, gebildet aus dem mit 100 multiplizierten Quotienten der Äquivalenzverhältnisse von Isocyanatgruppen und der Summe der Hydroxylgruppen aus der Polyolkombination und dem Kettenverlängerer, muß 97 bis 99 betragen.

Diese Polyurethanformmasse kann problemlos zu einer homogenen Schmelze erhitzt werden, aus welcher molekular gleichmäßige Filamente erspinnbar sind. Die Spinnbarkeit wird begünstigt durch die oberhalb des Schmelzpunktes niedrigviskose Schmelze.

Die sehr elastischen Filamente sind aufgrund des guten Kristallationsverhaltens der Schmelze zu Vliesstoffen mit hohen Festigkeiten bei niedrigem Flächengewicht verarbeitbar; die auf den Vliesstoff übertragenen elastischen Eigenschaften machen diesen für die Fertigung von Inkontinenzartikeln äußerst geeignet.

Der besondere und nicht zu erwartende Vorteil der Formmasse ist die schnelle und vollständige biologische Abbaubarkeit durch Enzyme, wobei aufgrund der rein aliphatischen Bausteine auch beim Abbau nur aliphatische und damit nicht toxische Zwischenprodukte entstehen.

Bereits bei 30% relativer Feuchtigkeit und 23°C erfolgt im Erd-Enzym-Medium eine sehr rasche Aufspaltung der Polyurethankette durch Hydrolyse der Polyestersegmente. In einem weiteren Abbauschritt werden die Polyurethanbruchstücke durch Enzyme vollständig zerlegt. Ursache dafür scheinen die hydrophilen Gruppen im Polyurethan-Weichsegment der erfindungsgemäßen Formmasse zu sein.

Bei Erdeingrabungsversuchen von Vliesstoffen, deren Filamente aus der erfindungsgemäßen Polyurethan-Formmasse hergestellt wurden, zeigte sich unter den obigen Bedingungen bereits nach wenigen Tagen eine Minderung der Festigkeit, anschließend eine Verfärbung mit Lochfraß und - nach acht Wochen - die vollständige Auflösung der Vliesstoffe.

Abbauversuche mit vergleichbaren aliphatischen Polyester-Polyurethanen ohne hydrophile Polyethergruppen im Polyurethan-Weichsegment ergaben erst nach vier Monaten eine signifikante Reduzierung der Festigkeitseigenschaften, ohne daß dabei ein deutlicher Abbau des Polyurethans erfolgt wäre.

Wegen der gewählten NCO-Kennzahl von 97 - 99, also Isocyanat im Unterschuß, und infolge der Herstellung der Formmasse im Einstufen-Verfahren werden Nebenreaktionen ausgeschlossen, die zu gelartigen und teilverzweigten Polymeranteilen führen, und ein einwandfreier Schmelzefluß für den Spinnprozeß erzielt.

Die nachfolgenden, beispielhaften Rezepturen ergeben besonders günstige Schmelzeigenschaften der Polyurethan-Formmasse:

### Beispiel 1

Zusammensetzung der Mischung:
90,0 Gew.-Teile Polyesterdiol auf Basis Adipinsäure und Ethandiol
10,0 Gew.-Teile Polyethylenglykol mit einem MG von 2000
11,88 Gew.-Teile 1,4-Butandiol
30,0 Gew.-Teile 1,6-Hexamethylendiisocyanat

Schmelzeigenschaften der Formmasse:
Fp: 170°C
Schmelzindex nach DIN 53735 bei 2,16 kg Belastung,
   gemessen bei 180°C: 32 g/10 min
   190°C: 95 g/10 min
   200°C: 175 g/10 min

### Beispiel 2

Zusammensetzung der Mischung:
90,0 Gew.-Teile Polyesterdiol auf Basis Adipinsäure und Ethandiol
10,0 Gew-.Teile Polyethylenglykol mit einem MG von 800
11,2 Gew.-Teile 1,4-Butandiol
30,0 Gew.-Teile 1,6-Hexamethylendiisocyanat

Schmelzeigenschaften der Formmasse:
FP: 160°C
Schmelzindex nach DIN 53735 bei 2,16 kg Belastung,
   gemessen bei 170 : 42 g/10 min
   180: : 79 g/10 min
   190 : 137 g/10 min

## Patentansprüche

1. Thermoplastische Polyurethan-Formmasse, erhältlich durch Umsetzung von 100 Gew.-Teilen eines Polyolgemischen mit 1,6-Hexamethylendiisocyanat, Isophorondiisocyanat oder Dicyclohexylmethan-4,4'-Diisocyanat -Diisocyanat und Diol-Kettenverlängerungsmittel, wobei die NCO-Kennzahl, gebildet aus dem mit 100 multiplizierten Quotienten der Äquivalenzverhältnisse von Isocyanatgruppen und der Summe der Hydroxylgruppen von Polyolgemisch und Kettenverlängerungsmittel, 97 bis 99 beträgt, dadurch gekennzeichnet, daß das Polyolgemisch aus 70 bis 90 Gew.-Teilen Polyesterpolyol mit einem Molekulargewicht von 2000 auf Basis Adipinsäure mit Ethandiol, Butandiol, Hexandiol, Diethylenglykol oder Neopentylglykol sowie aus 10 bis 30 Gew.-Teilen eines Polyethylenglykols mit einem Molekulargewicht von 800 bis 4000 besteht, daß das Kettenverlängerungsmittel 1,4-Butandiol und/oder 1,6-Hexandiol ist und daß das 1,6-Hexamethylendiisocyanat, Isophorondiisocyanat oder Dicylohexylmethan-4,4'-Diisocyanat in einem Äquivalenzverhältnis zum Polyolgemisch von 2,8:1,0 bis 12,0:1,0 vorliegt, das Kettenverlängerungsmittel in einem Äquivalenzverhältnis zum Polyolgemisch von 1,75:1,0 bis 11,3:1,0.

2. Thermoplastische Filamente, ersponnen aus einer Polyurethan-Formmasse mit den Kennzeichen des Anspruchs 1.

## Claims

1. A thermoplastic polyurethane moulding material obtainable by reaction of 100 parts by weight of a polyol mixture with 1,6-hexamethylene diisocyanate, isophorone diisocyanate or dicyclohexylmethane 4,4'-diisocyanate and a diol chain extender, the NCO number, defined as 100 times the quotient of the equivalence ratios of isocyanate groups and the sum of the hydroxyl groups in the polyol mixture and the chain extender, being in the range from 97 to 99, characterized in that the polyol mixture is composed of from 70 to 90 parts by weight of a polyesterpolyol which has a molecular weight of 2000 and is based on adipic acid combined with ethanediol, butanediol, hexanediol, diethylene glycol or neopentylglycol and from 10 to 30 parts by weight of polyethylene glycol having a molecular weight of from 800 to 4000, in that the chain extender is 1,4-butanediol and/or 1,6-hexanediol, and in that the 1,6-hexamethylene diisocyanate, isophorone diisocyanate or dicyclohexylmethane 4,4'-diisocyanate is present in an equivalence ratio to the polyol mixture of from 2.8:1.0 to 12.0:1.0 and the chain extender is present in an equivalence ratio to the polyol mixture of from 1.75:1.0 to 11.3:1.0.

2. Thermoplastic filaments spun from a polyurethane moulding material having the characterizing features of claim 1.

## Revendications

1. Matériau de moulage de polyuréthane thermoplastique que l'on peut obtenir par réaction de 100 parties en poids d'un mélange polyol avec du 1,6-hexaméthylène diisocyanate de l'isophorone diisocyanate ou du dicyclohexylméthane-4,4'-diisocyanate et d'un élément d'allongement de chaîne diol, le nombre caractéristique NCO formé avec le quotient multiplié par 100 des rapports d'équivalence des groupes isocyanate et de la somme des groupes hydroxyle de mélange polyol et de l'élément d'allongement de chaîne étant de 97 à 99, caractérisé en ce que le mélange polyol est composé de 70 à 90 parties en poids de polyesterpolyol d'un poids moléculaire de 2000 à base d'acide adipique avec de l'éthanediol, du butanediol, de l'hexanediol, du diéthylèneglycol ou de néopentylglycol ainsi que de 10 à 30 parties en poids d'un polyéthylèneglycol avec un poids moléculaire de 800 à 4000, en ce que l'élément d'allongement de chaîne est du 1,4-butanediol et/ou du 1,6-hexanediol et en ce que le 1,6-hexaméthylène diisocyanate, l'isophorone diisocyanate ou le dicyclohexylméthane-4,4'-diisocyanate est présent dans un rapport d'équivalence avec le mélange polyol de 2,8:1,0 à 12,0:1,0, l'élément d'allongement de chaîne dans un rapport d'équivalence avec le mélange polyol de 1,75:1,0 à 11,3:1,0.

2. Filaments thermoplastiques filés à partir d'un matériau de moulage de polyuréthane avec les caractéristiques décrites à la revendication 1.
